# EUROPEAN PATENT APPLICATION

(11) **EP 4 166 150 A1**
(43) Date of publication of application: **19.04.2023**
(21) Application number: 21306448.8
(22) Date of filing: 15.10.2021
(51) Int. Cl.: A61K 35/741, A61K 45/06, C07K 16/18, C12N 1/20, A61K 39/00, A61P 35/00

(54) **ASSOCIATION OF A FAECALIBACTERIUM PRAUSNITZII STRAIN AND ANTI-PD-1, ANTI-PD-L1 OR ANTI-CTLA-4 ANTIBODIES FOR THE TREATMENT OF CANCER**

(71) Applicant: Exeliom Biosciences, 21000 Dijon (FR)
(72) Inventor: RUFFIE, Pauline, 33400 Talence (FR); HADIDA, Benjamin, 75011 Paris (FR); SOKOL, Harry, 75015 Paris (FR)
(74) Representative: Cabinet Nony

(57) **Abstract**

The present invention relates to an association of a *Faecalibacterium prausnitzii* bacterial strain deposited to the CNCM under the accession number 1-4573; and at least one immune checkpoint inhibitor, in particular at least one monoclonal antibody selected from the group consisting of anti-PD-1, anti-PD-Ll and anti-CTLA-4 monoclonal antibodies for its use for preventing and/or treating a cancer in a mammal patient. The association as such is also considered.

## Description

### Field of the Invention

The present invention relates to the field of immunology and more particularly to an association of a *Faecalibacterium prausnitzii* bacterial strain and immune checkpoint inhibitors, and in particular of the *Faecalibacterium prausnitzii* bacterial strain deposited to the CNCM under the accession number I-4573 and anti-PD-1, anti-PDL-1 and/or anti-CTLA-4 monoclonal antibodies, and their use in human medicine.

In particular, the present invention relates to the use of an association of a *Faecalibacterium prausnitzii* bacterial strain and immune checkpoint inhibitors, and in particular of the *Faecalibacterium prausnitzii* bacterial strain deposited to the CNCM under the accession number I-4573 and anti-PD-1, anti-PD-L1 and/or anti-CTLA-4 monoclonal antibodies in the treatment of cancers, in particular anti-PD-1 and/or anti-PD-L1 monoclonal antibodies.

### Description of Related Art

Cancer ranks as the first or second leading cause of death. Worldwide, an estimated 19.3 million new cancer cases and almost 10 million cancer deaths occurred in 2020. The global cancer burden is expected to be 28.4 million cases in 2040, a 47% rise from 2020, with a larger increase in transitioning versus transitioned countries due to demographic changes. Inaccessible or non-sufficiently active treatment are common in this filed. There is thus a well-recognized need to develop new and improved therapies for treating cancers.

Immunotherapies represent a real hope in the treatment of a great number of cancers, in particular in the treatment of cancers not currently efficiently cured by conventional therapies. This treatment mainly consists in the administration of monoclonal antibodies directed against the tumoral cells or directed against activators or inhibitors of a checkpoint of the immune response against cancers (also termed ICI for Immune Checkpoint Inhibitors). In particular, tremendous advances have been made in the treatment of cancers by using ICI targeting the cytotoxic T lymphocyte-associated antigen 4 (CTLA-4), the programmed cell death protein (PD-1) or the Programmed death-ligand 1 (PDL-1).

However, response rate remains relatively low in most cases. Indeed, depending on the cancer considered, from 40% to 95% of the patients are resistant to treatments based on ICIs. The escaping mechanisms are complex and are currently strongly studied by the scientific community.

Thus, there remains a need in the art for the provision of an effective and improved therapy for treating cancers.

There is in particular a need in the art for the provision of a mean to improve the efficacy of immune checkpoint inhibitors (ICI), and in particular a mean to increase their efficacy against tumors resisting to such treatment.

### Summary of the invention

The present invention in particular relates to the following items:
**Item 1**: an association of:
- a *Faecalibacterium prausnitzii* bacterial strain deposited to the CNCM under the accession number 1-4573; and
- at least one immune checkpoint inhibitor, in particular at least one monoclonal antibody selected from the group consisting of anti-PD-1, anti-PD-L1 and anti-CTLA-4 monoclonal antibodies,
for its use for preventing and/or treating a cancer in a mammal patient.

The inventors indeed show for the first time that the *in vivo* administration of the *Faecalibacterium prausnitzii* bacterial strain deposited to the CNCM under the accession number I-4573 is able to restore the effectiveness of an immune checkpoint inhibitor against a tumor in an individual.

In particular, the inventors firstly confirmed that the alteration of the microbiota of an individual having a tumor by administration of antibiotics compromises the anti-tumoral effect of an ICI treatment, and in particular of an anti-PDL-1 treatment (Routy et al., Science 359, 91-97 (2018)) and serves as an appropriate model of non-responders to single-agent ICI therapy (i.e. individual whose tumor is resistant to treatments based on ICIs). On this basis, the inventors have surprisingly identified that the administration of the *Faecalibacterium prausnitzii* bacterial strain deposited to the CNCM under the accession number I-4573 to the said individual significantly restores the efficacy of the anti-PDL-1.

Such results are highly unexpected considering that the *Faecalibacterium prausnitzii* specifically implemented in the present invention, i.e. the *Faecalibacterium prausnitzii* bacterial strain deposited to the CNCM under the accession number I-4573, has been known until now for its anti-inflammatory properties (WO2017129515). On the contrary, in the present invention, it is demonstrated for the first time the ability of this strain to potentiate the immuno-stimulatory properties of an immune checkpoint inhibitor in an individual in need thereof.

Recent studies have demonstrated that the gut microbiome is able to modulate the response to anti-PD-1 immunotherapies in melanoma patients and that fecal microbiota transplantation (FMT) with stools from responders to anti-PD-1 therapy were able to improve the response of the administered mice to anti-PD-L1 therapy (Gopalakrishnan et al. Science 359, 97-103 (2018); Davar et al., Science 371, 595-602 (2021) et Baruch et al. Science 10.1126/science.abb5920 (2020)). None of these studies were able to determine the actual mechanisms of action leading to this result, and in particular which one of the numerous bacteria present in the stools were responsible for this improved response and which one were pure biomarkers providing some insight as to the chances that a given patient will be responsive or not to an ICI therapy. Another problem linked to these studies, and identified by them, is that the administration of a stool, in particular of a stool from a responder to anti-PD-1 / anti-PD-L1 therapy, is not reproductible as its composition, and in particular the number and variety of bacteria contained in it, will unpredictably (i) vary from one donor to another and (ii) even vary over time in the same donor. Besides, the shift of the patient's microbiota composition following faecal microbiota transplant towards donor composition may not maintain over time, especially after antibiotic exposure.

Accordingly, the inventors managed to identify, for the first time, a new, effective and perfectly safe way to improve the efficacy of immune checkpoint inhibitors, and in particular a mean to increase their efficacy against tumors resisting to such treatment.
**Item 2**: The association for its use according to item 1, wherein the immune checkpoint inhibitor is an anti-PD-L1 monoclonal antibody or an anti-PD-1 monoclonal antibody, in particular an anti-PD-L1 monoclonal antibody.
**Item 3**: The association for its use according to item 1 or 2, wherein the said mammal patient is selected from the group consisting of primates and human beings.
**Item 4:** The association for its use according to any one of items 1 to 3, wherein the said mammal patient is a human being.
**Item 5:** The association for its use according to any one of the preceding items, wherein the said mammal patient has an intestinal microbial dysbiosis.
**Item 6:** The association for its use according to any one of the preceding items, wherein the cancer is a solid or liquid tumor, and in particular is selected from the group consisting of fibrosarcoma, bladder cancer, breast cancer, cervix cancer, colorectal cancer, kidney cancer, lung cancer, lymphoma, leukemia, myeloma, melanoma, neuroblastoma, a Wilms tumor, oral or oropharyngeal cancer, pancreatic cancer, prostate cancer, retinoblastoma, thyroid cancer, uterine cancer, adenoid cystic carcinoma, adrenocortical tumor, chondrosarcoma, desmoid tumor, desmoplastic small round cell tumor, endocrine tumor, endodermal sinus tumor, epithelioid hemangioendothelioma, ewing sarcoma, nephroma, adrenal gland tumor, amyloidosis, anal cancer, appendix cancer, cholangiocarcinoma, glioma, non-rhabdomyosarcoma soft tissue sarcoma (NRSTS), paraspinal sarcoma, renal cell carcinoma, rhabdomyosarcoma, synovial sarcoma, bone cancer, brain cancer, central nervous system tumors, cervical cancer, esophageal cancer, eye cancer, eyelid cancer, gastrointestinal cancer, HIV/AIDS-related cancer, lacrimal gland cancer, laryngeal or hypopharyngeal cancer, leukemia, liver cancer, meningioma, nasopharyngeal cancer, ovarian cancer, fallopian tube cancer, peritoneal cancer, parathyroid cancer, penile cancer, salivary gland cancer, sarcoma, non-melanoma skin cancer, small bowel cancer, stomach cancer, testicular cancer, thymoma and thymic carcinoma, vaginal cancer and vulvar cancer.
**Item 7:** The association for its use according to any one of the preceding items, wherein the cancer is a fibrosarcoma.
**Item 8:** The association for its use according to any one of the preceding items, wherein the said *Faecalibacterium prausnitzii* bacterial strain deposited to the CNCM under the accession number I-4573 and the said at least one immune checkpoint inhibitor, in particular at least one monoclonal antibody selected from the group consisting of anti-PD-1, anti-PD-L1 and anti-CTLA-4 monoclonal antibodies, are administered to the mammal patient in the same composition or in separate compositions, preferably in separate compositions, said composition(s) further comprising a physiologically acceptable medium.
**Item 9:** The association for its use according to any one of the preceding items, wherein the association further comprises at least one additional anticancer drug different from the said *Faecalibacterium prausnitzii* bacterial strain deposited to the CNCM under the accession number I-4573 and from an anti-PD-1, anti-PD-L1 or anti-CTLA-4 monoclonal antibody.
**Item 10:** The association for its use according to item 9, wherein the at least one additional anticancer drug is selected from the group consisting of monoclonal or bi-specific antibodies, in particular selected from the group consisting of:
   - anti-CD137, anti-TIM-3, anti-B7-H3, anti-CD134, anti-CD154, anti-LAG-3, anti-CD227, anti-BTNA3, anti-CD39, anti-CD73, anti-CD115, anti-SIRP alpha, anti-SIRP gamma, anti-CD28, anti-NCR, anti-NKp46, anti-NKp30, anti-NKp44, anti-NKG2D and anti-DNAM-1 monoclonal or bispecific antibodies; and
   - anti-CTLA-4/anti-PDL-1, anti-CTLA-4/anti-PD-1 and anti-PD-1/anti-PD-L1 bispecific antibodies.
**Item 11:** An association of:
   - a *Faecalibacterium prausnitzii* bacterial strain deposited to the CNCM under the accession number I-4573; and
   - at least immune checkpoint inhibitor, in particular at least one monoclonal antibody selected from the group consisting of anti-PD-1, anti-PD-L1 and anti-CTLA-4 monoclonal antibodies,
   the association being in particular as defined in any one of items 2, 3, 9 and 10.

### Brief description of the drawings

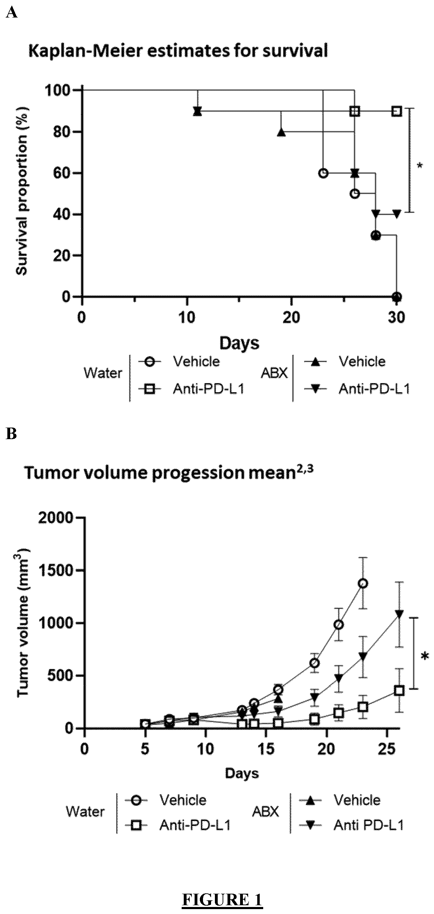

**Figure 1A**: represents the survival proportion (%) (represented as Kaplan-Meier plot) over 30 days of MCA-205 tumor (sarcoma) bearing mice exposed either to Vehicle (200µL of PBS/Glycerol by oral gavage, once a day, starting from day 6 post-tumor inoculation until the end of the study) (n=10) 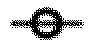 , to anti-PD-L1 antibody (5mg/kg, intraperitoneally, once a day, on days 6, 9, 12, and 15) (n=10) 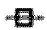 , to antibiotic (ABX) (starting from day -14 prior tumor-inoculation until the end of the study) (n=10) 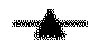 and to ABX (starting from day -14 prior tumor-inoculation until the end of the study) + anti-PD-L1 antibody (5mg/kg, intraperitoneally, once a day, on days 6, 9, 12, and 15) (n=10) 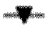 . Statistical analysis was based on the Mantel-Cox test.
   Ordinate: survival proportion (%).
   Abscissa: Days post tumor cell inoculation.
**Figure 1B**: represents the mean tumor volume evolution over 26 days of MCA-205 tumor (sarcoma) bearing mice exposed either to Vehicle (200µL of PBS/Glycerol by oral gavage, once a day, starting from day 6 post-tumor inoculation until the end of the study) (n=10) 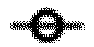 , to anti-PD-L1 antibody (5mg/kg, intraperitoneally, once a day, on days 6, 9, 12, and 15) (n=10) 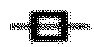 , to antibiotic (ABX) (starting from day -14 prior tumor-inoculation until the end of the study) (n=10) 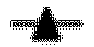 and to ABX (starting from day -14 prior tumor-inoculation until the end of the study) + anti-PD-L1 antibody (5mg/kg, intraperitoneally, once a day, on days 6, 9, 12, and 15) (n=10) 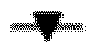 . Statistical analysis was based on the Mann-Whitney test.
   Ordinate: Tumor volume (mm³).
   Abscissa: Days post tumor cell inoculation.

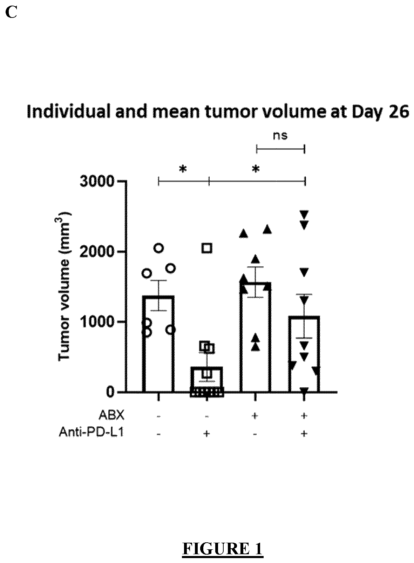

**Figure 1C**: represents the mean tumor volume at day 26 of MCA-205 tumor (sarcoma) bearing mice exposed either to Vehicle (200µL of PBS/Glycerol by oral gavage, once a day, starting from day 6 post-tumor inoculation until the end of the study) (n=10) 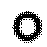 , to anti-PD-L1 antibody (5mg/kg, intraperitoneally, once a day, on days 6, 9, 12, and 15) (n=10) 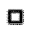 , to antibiotic (ABX) (starting from day -14 prior tumor-inoculation until the end of the study) (n=10) ▲ and to ABX (starting from day -14 prior tumor-inoculation until the end of the study) + anti-PD-L1 antibody (5mg/kg, intraperitoneally, once a day, on days 6, 9, 12, and 15) (n=10) ▼. Statistical analysis was based on the Mann-Whitney test.
   Ordinate: Tumor volume (mm³).
   Abscissa: group of mice considered as defined above, from left to right in the order indicated above.

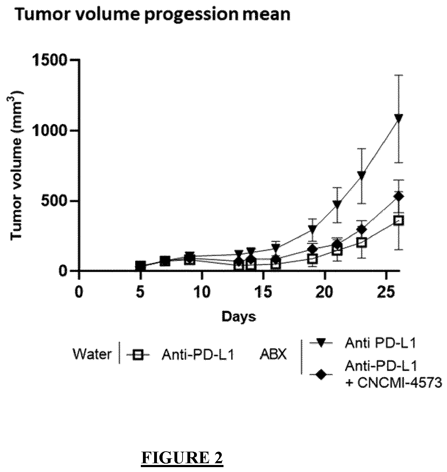

**Figure 2**: represents the mean tumor volume evolution over 26 days of MCA-205 tumor (sarcoma) bearing mice exposed either to anti-PD-L1 antibody (5mg/kg, intraperitoneally, once a day, on days 6, 9, 12, and 15) (n=10) 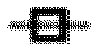 , to ABX (starting from day -14 prior tumor-inoculation until the end of the study) + anti-PD-L1 antibody (5mg/kg, intraperitoneally, once a day, on days 6, 9, 12, and 15) (n=10) 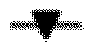 and to ABX (starting from day -14 prior tumor-inoculation until the end of the study) + anti-PD-L1 antibody (5mg/kg, intraperitoneally, once a day, on days 6, 9, 12, and 15) + strain CNCM I-4573 (200µL of EXL01 test bacteria in PBS/Glycerol by oral gavage, once a day (1×10¹⁰ TCC (Total Cell Counts) per day), starting from day 6 post-tumor inoculation until the end of the study) (n=10) 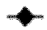 . Statistical analysis was based on the Mann-Whitney test.
   Ordinate: Tumor volume (mm³).
   Abscissa: Days post tumor cell inoculation.

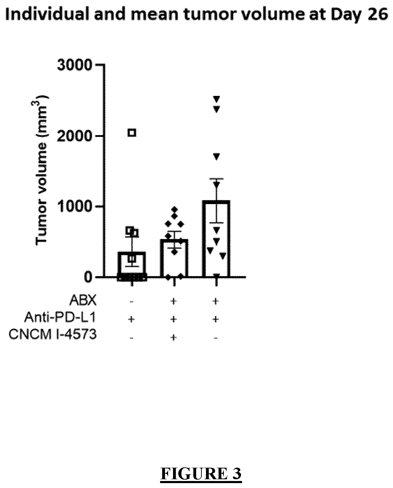

**Figure 3**: represents the individual and mean tumor volume at Day 26 MCA-205 tumor (sarcoma) bearing mice exposed either to anti-PD-L1 antibody (5mg/kg, intraperitoneally, once a day, on days 6, 9, 12, and 15) (n=10) 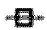 (left column), to ABX (starting from day -14 prior tumor-inoculation until the end of the study) + anti-PD-L1 antibody (5mg/kg, intraperitoneally, once a day, on days 6, 9, 12, and 15) + strain CNCM I-4573 (200µL of strain CNCM I-4573 in PBS/Glycerol by oral gavage, once a day (1×10¹⁰ TCC (Total Cell Counts) per day), starting from day 6 post-tumor inoculation until the end of the study) (n=10) 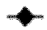 (column in the middle) or to ABX (starting from day -14 prior tumor-inoculation until the end of the study) + anti-PD-L1 antibody (5mg/kg, intraperitoneally, once a day, on days 6, 9, 12, and 15) (n=10) 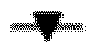 (right column). Statistical analysis was based on the Mann-Whitney test.
   Ordinate: Tumor volume (mm³).
   Abscissa: group of mice considered as defined above.

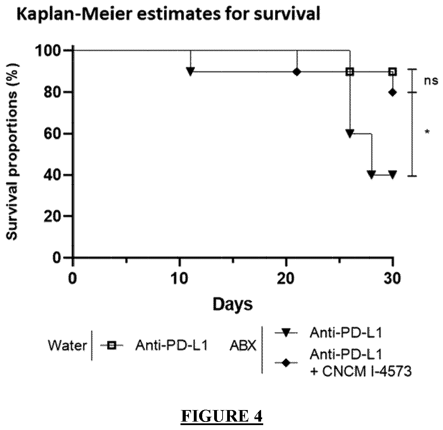

**Figure 4**: represents the survival proportion (%) (represented as Kaplan-Meier plot) over 30 days of MCA-205 tumor (sarcoma) bearing mice exposed either to anti-PD-L1 antibody (5mg/kg, intraperitoneally, once a day, on days 6, 9, 12, and 15) (n=10) 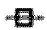 , to ABX (starting from day -14 prior tumor-inoculation until the end of the study) + anti-PD-L1 antibody (5 mg/kg, intraperitoneally, once a day, on days 6, 9, 12, and 15) (n=10) 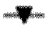 or to ABX (starting from day -14 prior tumor-inoculation until the end of the study) + anti-PD-L1 antibody (5 mg/kg, intraperitoneally, once a day, on days 6, 9, 12, and 15) + strain CNCM I-4573 (200µL of strain CNCM I-4573 in PBS/Glycerol by oral gavage, once a day (1×10¹⁰ TCC (Total Cell Counts) per day), starting from day 6 post-tumor inoculation until the end of the study) (n=10) 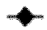 . Statistical analysis was based on the Mantel-Cox test.
   Ordinate: survival proportions (%).
   Abscissa: Days post tumor cell inoculation.

### Detailed description of the invention

### Definitions

In order for the invention to be more completely understood, several definitions are set forth below. Such definitions are meant to encompass grammatical equivalents.

The term "antibody" is used herein in the broadest sense. "Antibody" refers to any polypeptide which at least comprises (i) a Fc region and (ii) a binding polypeptide domain derived from a variable region of an immunoglobulin. Antibodies thus include, but are not limited to, full-length immunoglobulins, antibodies, antibody conjugates and fragments of each respectively. The terms "antibody" and "immunoglobulin" may be used interchangeably herein. The antibodies more particularly considered herein are monoclonal antibodies, in particular immune checkpoint inhibitors (ICI) monoclonal antibodies and more particularly monoclonal antibodies selected from the group consisting of anti-PD-1, anti-PD-L1 and anti-CTLA-4 monoclonal antibodies, in particular consisting of anti-PD-1 and anti-PD-L1 monoclonal antibodies.

The terms "monoclonal antibody" as used herein refer to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen. The modifier "monoclonal" is not to be construed as requiring production of the antibody by any particular method. For example, the monoclonal antibodies to be used in accordance with the invention may be made by the hybridoma method first described by Kohler et al, Nature 256:495 (1975), or may be made by recombinant DNA methods (see, e.g., U.S. Patent No. 4,816,567). The "monoclonal antibodies" may also be isolated from phage antibody libraries using the techniques described in Clackson et al, Nature 352:624-628 (1991) or Marks et al, J. MoI Biol. 222:581-597 (1991), for example.

The terms "bispecific antibody" or "bispecific antibodies (BsAbs)" refers to molecules that combine the antigen-binding sites of two antibodies within a single molecule. Thus, a bispecific antibody is able to bind two different antigens simultaneously. Methods to obtain such antibodies are well known by the man skilled in the art.

In the context of the present invention, the terms "*preventing*" and "*prevent*" denote the reduction to a lesser degree of the risk or of the probability of occurrence of a given phenomenon, that is to say, in the present invention, a cancer, in particular a cancer as detailed here-after.

As used herein, the terms "*treating*" or "*treat*" include alleviation of the symptoms associated with a specific disorder or condition and/or elimination of said symptoms, that is to say, in the present invention, a cancer, in particular a cancer as detailed here-after.

In the present invention, the term "*immune checkpoint inhibitor*" (also referred to as ICI) refers to a kind of immune modulating anticancer drug that target immune checkpoints, key regulators of the immune system that when stimulated can dampen the immune response to an immunologic stimulus. ICI can be in different forms, such as antibodies (monoclonal, bispecific, ...), RNAi molecules or antisense molecules.

By "*intestinal microbial dysbiosis*", it is intended in the present invention to designate as a significant deviation from a balanced microbiota, in terms of global microbiota profile, metabolism or levels of particular taxa.

A "*physiologically acceptable medium*" according to the invention means a carrier or excipient that is physiologically acceptable to the treated mammal patient while retaining the therapeutic properties of the compounds with which it is administered. One exemplary pharmaceutically acceptable medium is physiological saline. Other physiologically acceptable media in the case of the present invention are known to those skilled in the art.

The terms "*malignant cells*", "*cancer cells*" and "*tumor cells*" may be used interchangeably herein. As used herein, "tumor cells" refer to cells which hyperproliferate autonomously *in vivo.* Examples of tumor cells include cells included in (1) sarcomas such as osteosarcoma and soft tissue sarcoma, (2) carcinomas such as carcinoma of the breast, carcinoma of the lung, carcinoma of the bladder, carcinoma of the thyroid gland, carcinoma of the prostate, carcinoma of the colon, colorectal carcinoma, carcinoma of the pancreas, carcinoma of the stomach, carcinoma of the liver, carcinoma of the uterus, carcinoma of the cervix and carcinoma of the ovary, (3) lymphomas such as Hodgkin lymphoma and non-Hodgkin lymphoma, (4) neuroblastomas, (5) melanomas, (6) myelomas, (7) Wilms tumors, (8) leukemias such as acute myelocytic leukemia (AML), chronic myelocytic leukemia (CML), acute lymphocytic leukemia (ALL) and chronic lymphocytic leukemia (CLL), (9) gliomas, and (10) retinoblastomas.

As used herein, the term "*cancer*" means the uncontrolled, abnormal growth of cells and includes within its scope all the well-known diseases that are caused by the uncontrolled and abnormal growth of cells. Non-limiting examples of common cancers include fibrosarcoma, bladder cancer, breast cancer, ovarian cancer and gastric cancer, cervical cancer, colon cancer, endometrial cancer, head and neck cancer, lung cancer, melanoma, multiple myeloma, leukemia (e.g. myeloid, lymphocytic, myelocytic and lymphoblastic leukemias), non-Hodgkin's lymphoma, prostate cancer, rectal cancer, malignant melanomas, and in particular fibrosarcoma.

A cancer considered herein may in particular be a solid or liquid tumor.

In particular, cancer cells of the invention can be cells included in cancers selected from the group consisting of fibrosarcoma, bladder cancer, breast cancer, cervix cancer, colorectal cancer, kidney cancer, lung cancer, lymphoma, leukemia, myeloma, melanoma, neuroblastoma, a Wilms tumor, oral or oropharyngeal cancer, pancreatic cancer, prostate cancer, retinoblastoma, thyroid cancer, uterine cancer, adenoid cystic carcinoma, adrenocortical tumor, chondrosarcoma, desmoid tumor, desmoplastic small round cell tumor, endocrine tumor, endodermal sinus tumor, epithelioid hemangioendothelioma, ewing sarcoma, nephroma, adrenal gland tumor, amyloidosis, anal cancer, appendix cancer, cholangiocarcinoma, glioma, non-rhabdomyosarcoma soft tissue sarcoma (NRSTS), paraspinal sarcoma, renal cell carcinoma, rhabdomyosarcoma, synovial sarcoma, bone cancer, brain cancer, central nervous system tumors, cervical cancer, esophageal cancer, eye cancer, eyelid cancer, gastrointestinal cancer, HIV/AIDS-related cancer, lacrimal gland cancer, laryngeal or hypopharyngeal cancer, leukemia, liver cancer, meningioma, nasopharyngeal cancer, ovarian cancer, fallopian tube cancer, peritoneal cancer, parathyroid cancer, penile cancer, salivary gland cancer, sarcoma, non-melanoma skin cancer, small bowel cancer, stomach cancer, testicular cancer, thymoma and thymic carcinoma, vaginal cancer and vulvar cancer.

### Association according to the invention

With the view of responding to the aims mentioned above, and in particular in order to provide a new treatment against cancer, whose improved efficiency is not linked with any observable toxicity in the treated organism, the inventors have conceived an association of:
- a *Faecalibacterium prausnitzii* bacterial strain deposited to the Collection Nationale de Cultures de Microorganismes de l'Institut Pasteur de Paris (CNCM) on January 21, 2012 under the accession number I-4573; and
- at least one immune checkpoint inhibitor.

An immune checkpoint inhibitor (ICI) according to the invention may in particular be an antibody, in particular an anti-PD-1, anti-PD-L1 or anti-CTLA-4 antibody. More particularly, the at least one immune checkpoint inhibitor according to the invention may be a monoclonal antibody selected from the group consisting of anti-PD-1, anti-PD-L1 and anti-CTLA-4 monoclonal antibodies.

An anti-PD-1 monoclonal antibody according to the invention can for example be selected from the group consisting of Nivolumab, Pembrolizumab and Cemiplimab.

An anti-PD-L1 monoclonal antibody according to the invention can for example be selected from the group consisting of Atezolizumab, Avelumab and Durvalumab.

An anti-CTLA-4 monoclonal antibody according to the invention can for example be selected from the group consisting of ipilimumab, tremelimumab and quavonlimab.

In particular, the ICI of an association according to the invention may be an anti-PD-L1 or an anti-PD-1 monoclonal antibody, and in particular be an anti-PD-L1 monoclonal antibody.

A *Faecalibacterium prausnitzii* bacterial strain deposited to the CNCM under the accession number I-4573 according to an association of the invention may be in an isolated form. By "*isolated form*" according to the invention, it is meant in a form isolated from any fecal material, or intestinal sampling. Accordingly, a bacterial strain of the invention is not administered to a patient in the form of a Fecal Microbiota Transplant (FMT). Methods for isolating a bacteria from the gut microbiota are well known to the man skilled in the art, such as for example illustrated in Foditsch et al. (PLoS One. 2014; 9(12): e116465).

A mammal patient according to the invention may be selected from the group consisting of primates and human beings, and may in particular be a human being.

A mammal patient according to the invention may in particular have an intestinal microbial dysbiosis.

An association according to the invention may further comprise at least one additional anticancer drug different:
- from the said *Faecalibacterium prausnitzii* bacterial strain deposited to the CNCM under the accession number I-4573; and
- from an anti-PD-1, anti-PD-L1 or anti-CTLA-4 monoclonal antibody.

The at least one additional anticancer drug may in particular be selected from the group consisting of monoclonal or bi-specific antibodies, in particular selected from the group consisting of:
- anti-CD137, anti-TIM-3, anti-B7-H3, anti-CD134, anti-CD154, anti-LAG-3, anti-CD227, anti-BTNA3, anti-CD39, anti-CD73, anti-CD115, anti-SIRP alpha, anti-SIRP gamma, anti-CD28, anti-NCR, anti-NKp46, anti-NKp30, anti-NKp44, anti-NKG2D and anti-DNAM-1 monoclonal or bispecific antibodies; and
- anti-CTLA-4/anti-PDL-1, anti-CTLA-4/anti-PD-1 and anti-PD-1/anti-PD-L1 bispecific antibodies.

### Medical uses according to the invention

As above-mentioned, the present invention, according to one of its aspects, relates to an association of:
- a *Faecalibacterium prausnitzii* bacterial strain deposited to the CNCM under the accession number I-4573, more particularly in an isolated form; and
- at least one immune checkpoint inhibitor, in particular at least one monoclonal antibody selected from the group consisting of anti-PD-1, anti-PD-L1 and anti-CTLA-4 monoclonal antibodies,
for its use for preventing and/or treating a cancer in a mammal patient.

These ICI, and in particular these monoclonal antibodies, are described throughout the present specification.

In order to treat a patient in need thereof, such as above-mentioned, a therapeutically effective dose of the association according to the invention may be administered.

By "*therapeutically effective dose*" herein is meant a dose that produces the effects for which it is administered. The exact dose will depend on the purpose of the treatment and will be ascertainable by one skilled in the art using known techniques.

Dosages may range from 10³ to 10¹² colony forming units (CFU), more preferably from 10⁷ to 10¹¹ CFU as a medicament, for example as a daily dose equivalent to 10⁹ CFU.

Dosages may also range from 10⁴ to 10¹² total cell count (TCC), more preferably from 10⁸ to 10¹² TCC as a medicament, for example as a daily dose equivalent to 10¹⁰ TCC.

Dosages may also range from 0.001 to 100 mg of anti-PD-1 and/or anti-PD-L1 monoclonal antibodies according to the invention per kg of body weight (mg/kg) or greater, for example 0.1, 1.0, 5, 10, or 50 mg/kg of body weight.

The dosage and frequency of administration may be adapted depending on the host response as well as the frequency of injection owing to a better tolerance.

For illustrative purposes only, the frequency of administration of an association according to the invention could be a daily administration for 5 to 15 consecutive days. The administration can alternatively be every 2, 3, 4, 5, 6 or 7 days for a period of up to several months. A *Faecalibacterium prausnitzii* bacterial strain deposited to the CNCM under the accession number 1-4573 and an immune checkpoint inhibitor (ICI) of an association according to the invention may be administered simultaneously or separately, such as for example separately during the same day, or even separately with at least one, or even more, days between the administration of the bacterial strain and the administration of the ICI.

As is known in the art, adjustments for protein degradation, systemic versus localized delivery, as well as the age, body weight, general health, sex, diet, time of administration, drug interaction and the severity of the condition may be necessary, and is easily determined with routine experimentation by those skilled in the art.

Administration of the association of the invention may be done in a variety of ways, and in particular orally or rectally.

In a particular embodiment, an association according to the invention is in a form suitable for administration by rectal or oral route.

As mentioned above, the invention relates to an association of:
- a *Faecalibacterium prausnitzii* bacterial strain deposited to the CNCM under the accession number I-4573; and
- at least one immune checkpoint inhibitor, in particular at least one monoclonal antibody selected from the group consisting of anti-PD-1, anti-PD-L1 and anti-CTLA-4 monoclonal antibodies,
for its use for preventing and/or treating a cancer in a mammal patient.

The *Faecalibacterium prausnitzii* bacterial strain deposited to the CNCM under the accession number I-4573 and the at least one immune checkpoint inhibitor, in particular at least one monoclonal antibody selected from the group consisting of anti-PD-1, anti-PD-L1 and anti-CTLA-4 monoclonal antibodies, may be combined within one and the same composition, or may be used in the form of separate compositions, which may be administered simultaneously or sequentially.

In a particular embodiment, the *Faecalibacterium prausnitzii* bacterial strain deposited to the CNCM under the accession number I-4573 and the at least one immune checkpoint inhibitor, in particular at least one monoclonal antibody selected from the group consisting of anti-PD-1, anti-PD-L1 and anti-CTLA-4 monoclonal antibodies, are administered in separate compositions.

When the *Faecalibacterium prausnitzii* bacterial strain deposited to the CNCM under the accession number I-4573 and the at least one immune checkpoint inhibitor, in particular at least one monoclonal antibody selected from the group consisting of anti-PD-1, anti-PD-L1 and anti-CTLA-4 monoclonal antibodies, are administered in separate compositions, the compositions can be administered to the mammal patient simultaneously or separately through the same route of through different routes.

By simultaneously, it is understood that the compositions can be administered at the same moment or up to the same day or couple of days.

By separately, it is understood that the compositions can be administered with at least several days, for example at least two days of difference.

In some embodiments, a composition comprising a *Faecalibacterium prausnitzii* bacterial strain deposited to the CNCM under the accession number I-4573 and a composition of ICI, and in particular of anti-PD-1, anti-PD-L1 and/or anti-CTLA-4 monoclonal antibod(y)ies, according to the invention is(are) in liquid form.

In some of the embodiments, a composition of a *Faecalibacterium prausnitzii* bacterial strain deposited to the CNCM under the accession number 1-4573 and a composition of ICI, and in particular of anti-PD-1, anti-PD-L1 and/or anti-CTLA-4 monoclonal antibod(y)ies, according to the invention is(are) in a solid form, which includes a lyophilized form.

These compositions may be formulated according to standard methods such as those described in Remington: The Science and Practice of Pharmacy (Lippincott Williams & Wilkins; Twenty first Edition, 2005).

As previously indicated, the compositions mentioned above comprise a physiologically/pharmaceutically acceptable medium/excipient. The terms "physiologically acceptable" and "pharmaceutically acceptable" are used interchangeably in the present text.

The association of the invention may be administered with other therapies or therapeutics, including for example, antibiotics, cytotoxic chemotherapies, small molecules, other biologicals, radiation therapy, surgery, etc, which are different from the additional anticancer drug mentioned above and which is also different:
- from the said *Faecalibacterium prausnitzii* bacterial strain deposited to the CNCM under the accession number I-4573; and
- from an anti-PD-1, anti-PD-L1 or anti-CTLA-4 antibody.

According to a particular embodiment, the association according to the invention may further comprise as other therapeutics at least one immunosuppressive drug, such as glucocorticoids, cytostatics (Azathioprine, Methotrexate), antibodies or drugs acting on immunophilins (Cyclosporine, Tacrolimus, Rapamicin).

These other therapeutics can be administered to the mammal patient in the same composition as the *Faecalibacterium prausnitzii* bacterial strain deposited to the CNCM under the accession number I-4573 or the at least one immune checkpoint inhibitor, in particular at least one monoclonal antibody selected from the group consisting of anti-PD-1, anti-PD-L1 and anti-CTLA-4 monoclonal antibodies, or in a separate composition.

In particular, these other therapeutics are administered in a composition separate from the *Faecalibacterium prausnitzii* bacterial strain deposited to the CNCM under the accession number I-4573 or the at least one immune checkpoint inhibitor, in particular at least one monoclonal antibody selected from the group consisting of anti-PD-1, anti-PD-L1 and anti-CTLA-4 monoclonal antibodies.

In a particular embodiment, the present invention relates to the use of an association of:
- a *Faecalibacterium prausnitzii* bacterial strain deposited to the CNCM under the accession number I-4573; and
- at least one immune checkpoint inhibitor, in particular at least one monoclonal antibody selected from the group consisting of anti-PD-1, anti-PD-L1 and anti-CTLA-4 monoclonal antibodies;
for the preparation of a medicament for preventing and/or treating a cancer in a mammal patient.

In another embodiment, the present invention relates to a method for the prevention and/or treatment of a cancer in a mammal patient, comprising administering to the said mammal patient an association of:
- a *Faecalibacterium prausnitzii* bacterial strain deposited to the CNCM under the accession number I-4573; and
- at least one immune checkpoint inhibitor, in particular at least one monoclonal antibody selected from the group consisting of anti-PD-1, anti-PD-L1 and anti-CTLA-4 monoclonal antibodies.

The present invention also relates to an association of:
- a *Faecalibacterium prausnitzii* bacterial strain deposited to the CNCM under the accession number 1-4573; and
- at least one immune checkpoint inhibitor, in particular at least one monoclonal antibody selected from the group consisting of anti-PD-1, anti-PD-L1 and anti-CTLA-4 monoclonal antibodies,
for its use as a medicament.

The present invention is further illustrated by the examples below.

### EXAMPLES:

### Cancer cell lines and induction of tumors in mice

Sarcoma MCA-205 tumor cell line was cultured in vitro according to Provider's specifications, DMEM supplemented with 10% FBS, 1% Penicillin-Streptomycin, 1mM HEPES.

MCA-205 tumor cells (500 000 cells/100 µL/mouse) were subcutaneously inoculated in the right flank of immunocompetent C57Bl/6J mice (9 week-old female).

### Animal groups

The objective of this study was to evaluate the ability of the *Faecalibacterium prausnitzii* bacterial strain deposited to the CNCM under the accession number I-4573 to restore anti-tumor effect of anti-PD-L1 antibody in MCA-205 tumor-bearing mouse model with a dysregulated microbiota.

The microbiota dysbiosis was induced by antibiotics (ABX) administration.

On day -15 prior-tumor inoculation, mice were randomized according to their body weight and allocated to ABX-treated and ABX-untreated groups.

MCA-205 sarcoma cells were subcutaneously inoculated at day 0 by injecting cells in the flank of 9 weeks-old female C57BL/6 mice (Charles River Laboratories). On day 5 post-tumor inoculation, mice were randomized on the basis of tumor volume and allocated to vehicle and anti-PD-L1 +/- test bacteria-treated groups.

Treatments were applied as follows (10 mice per group):
- Group 1: No antibiotic (ABX-Free) and no treatment (Vehicle : 200µL of PBS/Glycerol by oral gavage, once a day, starting from day 6 post-tumor inoculation until the end of the study).
- Group 2: ABX-Free + Anti-PD-L1 + Vehicle-treated group : 200µL of PBS/Glycerol by oral gavage, once a day, starting from day 6 post-tumor inoculation until the end of the study (Day 42) ; and anti-PD-L1 antibody at 5mg/kg, intraperitoneally, once a day, on days 6, 9, 12, and 15.
- Group 3: Treated with antibiotics (ABX) and no treatment (Vehicle : Antibiotics combination solution in drinking water, starting from day -14 prior tumor-inoculation until the end of the study; and 200µL of PBS/Glycerol by oral gavage, once a day, starting from day 6 post-tumor inoculation until the end of the study).
- Group 4: Treated with antibiotics (ABX) + Anti-PD-L1 + Vehicle-treated group : Antibiotics combination solution in drinking water, starting from day -14 prior tumor-inoculation until the end of the study; anti-PD-L1 antibody at 5mg/Kg, intraperitoneally, once a day, on days 6, 9, 12, and 15 ; and 200µL of PBS/Glycerol by oral gavage, once a day, starting from day 6 post-tumor inoculation until the end of the study.
- Group 5: Treated with antibiotics (ABX) + Anti-PD-L1 + *Faecalibacterium prausnitzii* bacterial strain deposited to the CNCM under the accession number I-4573 -treated group : Antibiotics combination solution in drinking water, starting from day -14 prior tumor-inoculation until the end of the study ; anti-PD-L1 antibody at 5mg/Kg, intraperitoneally, once a day, on days 6, 9, 12, and 15 ; and 200µL (1×10¹⁰ TCC) of strain CNCM I-4573 in PBS/Glycerol by oral gavage, once a day, starting from day 6 post-tumor inoculation until the end of the study.

Starting from day 5 after tumor cell inoculation, all experimental animal groups, each consisting of 10 mice, were monitored 3 times per week until day 30 for the following parameters:
- Tumor size: measured by physical examination, 3 times per week
- Body weight: monitored once a week before tumor inoculation then 3 times per week
- Survival: represented in a Kaplan-Meier plot, 3 times per week

### Antibiotic treatment (ABX)

Antibiotic solution consisting of Ampicillin (Sigma - 1mg/mL), Streptomycin (Sigma - 5mg/mL) and Colistin (Carbosynth - 1mg/mL) were prepared in drinking water supplemented with 1% Sucrose (Sigma) on a weekly basis and refrigerated. Cage bottles were changed once a week.

### Tested bacteria of the invention

*Faecalibacterium prausnitzii* bacterial strain deposited to the CNCM under the accession number 1-4573 was prepared as ready-to-use in PBS/Glycerol. Treatment started on Day 6 post tumor inoculation and was maintained all along the study with treatment occurring once daily (including the week-ends).

Treatment was performed by oral gavage with 200µL per mouse and per day - corresponding to 10¹⁰ TCC per dose

### Anti-PD-L1

Anti-PD-L1 treatment was applied by intraperitoneally administering 100 µg (for a 20 g mouse) of anti-PD-L1 antibody (5 mg/Kg). Treatments were repeated 4 times, on days 6, 9, 12, and 15 after tumor cell inoculation. Anti-PD-L1 antibody (clone 10F.9G2 - BioXCell) was diluted in sterile PBS at a concentration of 1 mg/mL. The stock solution was then aliquoted (amount for 1 day treatment) and stored at 4°C.

### Example 1: Effects of ABX on anti-PD-L1 mediated response

The survival proportion over 30 days of MCA-205 tumor bearing mice according to groups 1, group 2, group 3 and group 4 has firstly been examined.

The results obtained are represented in Figure 1A.

After 30 days, while the percentage of surviving mice was 90% in group 2, this percentage was only 40% in group 4, demonstrating that alteration of the microbiota by antibiotic administration compromises anti-tumor effect of an anti-PD-L1 treatment.

This is also illustrated in Figures 1B and 1C, where the tumor volume of mice from the same four groups was measured.

The mean tumor volume of mice from group 4 (ABX+anti-PD-L1) was significantly higher than the one of mice from group 2 (Anti-PD-L1).

This result also illustrates the negative impact of microbiota alteration by antibiotics on the anti-tumoral properties of an anti-PD-L1 treatment.

### Example 2: Effects of the strain according to the invention on ABX mediated response

The tumor volume evolution over 26 days of MCA-205 tumor bearing mice from group 2, group 4 and group 5 has also been examined.

The results obtained are represented in Figures 2 and 3.

After 26 days, while the mean tumor volume of mice from group 4 was above 1000 mm³, the corresponding value in group 5 was statistically inferior, at only about 500 mm³, similar to the one observed for mice from group 2.

The survival progression of the tested mice has also been measured during the experiment.

The results obtained are represented in Figure 4.

After 30 days, only 40% of the individuals from group 4, treated with antibiotics (ABX) + Anti-PD-L1, survived. On the contrary, 90% of the individuals from group 5 were still alive after 30 days due to the administration of the strain according to the invention. This % is the same % as the one obtained with group 2 in Figure 1A.

The Survival progression is significantly improved when tumour bearing mice having a microbiota dysbiosis are treated with the strain according to the invention in combination with anti-PDL-1 compared to anti-PDL-1 only.

These results demonstrate the ability of the strain according to the invention to significantly improve the anti-tumoral efficacy of the anti-PD-L1 treatment.

These results accordingly illustrate the fact that the strain according to the invention has the potential to rescue the anti-tumoral efficacy of ICI in non-responders.

## Claims

1. An association of:
- a *Faecalibacterium prausnitzii* bacterial strain deposited to the CNCM under the accession number I-4573; and
- at least one immune checkpoint inhibitor, in particular at least one monoclonal antibody selected from the group consisting of anti-PD-1, anti-PD-L1 and anti-CTLA-4 monoclonal antibodies,
for its use for preventing and/or treating a cancer in a mammal patient.

2. The association for its use according to claim 1, wherein the immune checkpoint inhibitor is an anti-PD-L1 monoclonal antibody or an anti-PD-1 monoclonal antibody, and is in particular an anti-PD-L1 monoclonal antibody.

3. The association for its use according to claim 1 or 2, wherein the said mammal patient is selected from the group consisting of primates and human beings.

4. The association for its use according to any one of claims 1 to 3, wherein the said mammal patient is a human being.

5. The association for its use according to any one of the preceding claims, wherein the said mammal patient has an intestinal microbial dysbiosis.

6. The association for its use according to any one of the preceding claims, wherein the cancer is a solid or liquid tumor, and in particular is selected from the group consisting of fibrosarcoma, bladder cancer, breast cancer, cervix cancer, colorectal cancer, kidney cancer, lung cancer, lymphoma, leukemia, myeloma, melanoma, neuroblastoma, a Wilms tumor, oral or oropharyngeal cancer, pancreatic cancer, prostate cancer, retinoblastoma, thyroid cancer, uterine cancer, adenoid cystic carcinoma, adrenocortical tumor, chondrosarcoma, desmoid tumor, desmoplastic small round cell tumor, endocrine tumor, endodermal sinus tumor, epithelioid hemangioendothelioma, ewing sarcoma, nephroma, adrenal gland tumor, amyloidosis, anal cancer, appendix cancer, cholangiocarcinoma, glioma, non-rhabdomyosarcoma soft tissue sarcoma (NRSTS), paraspinal sarcoma, renal cell carcinoma, rhabdomyosarcoma, synovial sarcoma, bone cancer, brain cancer, central nervous system tumors, cervical cancer, esophageal cancer, eye cancer, eyelid cancer, gastrointestinal cancer, HIV/AIDS-related cancer, lacrimal gland cancer, laryngeal or hypopharyngeal cancer, leukemia, liver cancer, meningioma, nasopharyngeal cancer, ovarian cancer, fallopian tube cancer, peritoneal cancer, parathyroid cancer, penile cancer, salivary gland cancer, sarcoma, non-melanoma skin cancer, small bowel cancer, stomach cancer, testicular cancer, thymoma and thymic carcinoma, vaginal cancer and vulvar cancer.

7. The association for its use according to any one of the preceding claims, wherein the cancer is a fibrosarcoma.

8. The association for its use according to any one of the preceding claims, wherein the said *Faecalibacterium prausnitzii* bacterial strain deposited to the CNCM under the accession number I-4573 and the said at least immune checkpoint inhibitor, in particular at least one monoclonal antibody selected from the group consisting of anti-PD-1, anti-PD-L1 and anti-CTLA-4 monoclonal antibodies, are administered to the mammal patient in the same composition or in separate compositions, preferably in separate compositions, said composition(s) further comprising a physiologically acceptable medium.

9. The association for its use according to any one of the preceding claims, wherein the association further comprises at least one additional anticancer drug different from the said *Faecalibacterium prausnitzii* bacterial strain deposited to the CNCM under the accession number I-4573 and from an anti-PD-1, anti-PD-L1 or anti-CTLA-4 monoclonal antibody.

10. The association for its use according to claim 9, wherein the at least one additional anticancer drug is selected from the group consisting of monoclonal or bi-specific antibodies, in particular selected from the group consisting of:
- anti-CD137, anti-TIM-3, anti-B7-H3, anti-CD134, anti-CD154, anti-LAG-3, anti-CD227, anti-BTNA3, anti-CD39, anti-CD73, anti-CD115, anti-SIRP alpha, anti-SIRP gamma, anti-CD28, anti-NCR, anti-NKp46, anti-NKp30, anti-NKp44, anti-NKG2D and anti-DNAM-1 monoclonal or bispecific antibodies; and
- anti-CTLA-4/anti-PDL-1, anti-CTLA-4/anti-PD-1 and anti-PD-1/anti-PD-L1 bispecific antibodies.

11. An association of:
- a *Faecalibacterium prausnitzii* bacterial strain deposited to the CNCM under the accession number I-4573; and
- at least one immune checkpoint inhibitor, in particular at least one monoclonal antibody selected from the group consisting of anti-PD-1, anti-PD-L1 and anti-CTLA-4 monoclonal antibodies,
the association being in particular as defined in any one of claims 2, 3, 9 and 10.
